Europäisches Patentamt

European Patent Office

Office européen des brevets

(11) Publication number: **0 455 326 A2**

(12) **EUROPEAN PATENT APPLICATION**

(21) Application number: **91301758.8**

(22) Date of filing: **04.03.91**

(51) Int. Cl.⁵: **A61L 11/00**

(30) Priority: **19.04.90 US 510700**

(43) Date of publication of application:
**06.11.91 Bulletin 91/45**

(84) Designated Contracting States:
**DE FR GB IT NL**

(71) Applicant: **WINFIELD CORPORATION**
**9750 Distribution Avenue**
**San Diego, California 92121(US)**

(72) Inventor: **Meijer, Robert S.**
**9052 Geraldine Place**
**San Diego, California 92123(US)**

(74) Representative: **Coxon, Philip et al**
**Eric Potter & Clarkson St. Mary's Court St.**
**Mary's Gate**
**Nottingham NG1 1LE(GB)**

(54) **Bag with disinfectant for sterilizing infectious waste.**

(57) A bag for use in sterilizing infectious waste has a flexible container which is formed with a closed end and a closeable open end. A pouch is attached to the container. Inside the pouch is a medium which has both granulate disinfectant constituents and pigment constituents. In one embodiment, the pouch is formed by bonding together two opposite portions of the container to form a seam which defines the boundary between the pouch and the container. In an alternative embodiment, the pouch is formed by bonding the edges of a separate sheet of flexible material to the container. If desired, the bag may be visually coded, such as by color-coding or bar-coding, to identify the bag as an infectious waste bag having an associated disinfectant medium.

Fig. 1

## FIELD OF THE INVENTION

The present invention relates generally to systems and apparatus which are used for decontaminating infectious waste. More particularly, the present invention is useful as a container for collecting infectious waste and conveying the waste to appropriate machinery for subsequent decontamination. The present invention is particularly, though not exclusively, useful for holding both the infectious waste and a predetermined amount of granulated disinfectant for decontaminating the waste during subsequent processing through appropriate machinery.

## BACKGROUND OF THE INVENTION

The disposal of infectious waste from hospitals and other medical establishments is a major problem. Indeed, the importance of proper and effective infectious waste disposal has become of greater concern in recent years, due to increased awareness of health problems such as the AIDS epidemic. In part because of the AIDS epidemic, definitions of what constitutes "infectious waste" are being broadened. Thus, the volume of infectious waste which must be disposed of is inceasing. Accordingly, the need for a system or apparatus which will accomplish the safe, efficacious, and cost effective disposal of significant volumes of infectious waste is growing.

One method for sterilizing and disposing of infectious waste involves incineration, wherein the waste is burned and the decontaminated ashes are properly disposed of. An alternative waste disposal method is to sterilize the waste in a steam autoclave or ethylene oxide autoclave prior to waste disposal. While effective for their intended purposes, both incinerators and autoclaves present ancillary waste disposal problems. Incinerators, for example, are difficult and costly to construct and are relatively expensive to maintain in an environmentally safe manner. Autoclaves too, present additional problems, such as the need to monitor the processed waste for 100% microbial kill. Additionally, waste which has been sterilized by autoclaving typically requires further disposal procedures, such as incineration, prior to final disposition of the waste in such places as ordinary landfills.

With the above discussion in mind, alternative infectious waste disposal systems have been proposed which disinfect waste which has been mechanically shredded into small particles. After the shredding process, a mist of disinfectant solution, typically a chlorine compound, is sprayed onto the shredded waste in order to sterilize the waste. The resulting slurry effluent and its decontaminated liquid constituents may then be separated from the solid constituents of the effluent and disposed of in an ordinary waste disposed system. Then, the decontaminated solid constituents of the effluent may be disposed of in ordinary landfills.

Unfortunately, decontamination of waste using liquified chlorine presents certain technical complications. First, liquified disinfectant loses its disinfectant potency during prolonged storage. Thus, there is a need to use liquified disinfectant that is relatively "fresh" in order to achieve an acceptable degree of waste decontamination. Second, it is relatively difficult to ensure that an appropriate amount of the disinfectant has been sprayed onto the waste during the disposal process. This is because an appropriate amount of disinfectant must contact the waste in order for the decontamination process to be efficacious. It is also important, however, to avoid spraying too much disinfectant into the disposal chamber of the sterilization apparatus, in order to avoid certain undesirable results, such as release of toxic gasses. The present invention recognizes that precise amounts of a dry chlorine compound which can be stored for a relatively lengthy period without losing its potency can be mixed with moistened waste in systems that mechanically destroy the waste to sterilize the infectious waste during the destruction process.

Accordingly, it is an object of the present intention to provide a waste disposal bag in which infectious waste may be discarded. It is a further object of the present invention to provide a waste disposal bag in which precise amounts of a dry chlorine-containing medium can be stored apart from the infectious waste held within the bag. Yet another object of the present invention is to provide a waste disposal bag which is destructable in a mechanical blender and which releases the chlorine-containing medium onto the waste during the destruction process. Finally, it is an object of the present invention to provide a waste disposal bag which is easy to use and comparatively cost-effective to manufacture.

A preferred embodiment of the invention provides a bag for use in the decontamination of infectious waste, which comprises:

means for surroundingly holding said waste; and

means for attaching a medium having disinfectant constituents to said holding means for release of said medium onto said waste when said bag is destroyed.

A bag for use in decontaminating infectious waste in accordance with the invention has a flexible container which forms a void for holding the waste. The container has a closed end and an open end which can be closed, such as by sealing, to hold the waste inside the container. Additionally, the container may be color coded or bar coded to identify the container as an infectious waste container. To decontaminate the waste upon destruction of the bag, a medium which contains a predetermined amount of disinfectant constitutents is contained in an enclosed pouch and the pouch, in turn is attached to the container. Preferably, the medium contains a solid granulated form of sodium hypochlorite. In addition to the disinfectant constituents, the medium may also contain pH-adjusting constituents, deodorant constituents, surfactants and a pigment for staining the waste during the decontamination process to eventualy identify the waste as decontaminated.

In the preferred embodiment of the present invention, the pouch is formed by bonding a portion of one side of the container to a portion of the opposite side of the container to thereby form a seam which defines the boundary between the pouch and the container. Alternatively, the pouch may be formed by bonding the edges of a separate sheet to the container. In either embodiment, the pouch forms an enclosed cavity in which the disinfectant-containing medium can be held.

To decontaminate infectious waste, the waste is first placed within the container. Then, the open end of the container is closed, such as by sealing, and the bag is placed into a machine, such as an industrial blender, which destroys the bag by pulverizing the bag. As the bag is destroyed, the integrity of the pouch is also compromised to release the disinfectant-containing medium onto the waste. As the bag is destroyed a liquid mist may be sprayed onto the blended materials and the medium to form a disinfectant solution with the disinfectant constituents of the medium to efficaciously blend with and decontaminate the waste. Finally, to identify the waste as being disinfected waste, the pigment contained in the medium is also mixed with the liquid mist to form a liquified pigment which stains the waste.

The novel features of this invention, as well as the invention itself, both as to its structure and its operation, will be best understood from the accompanying drawings, taken in conjunction with the accompanying description, in which similar reference characters refer to similar parts, and in which:

## BRIEF DESCRIPTION OF THE DRAWINGS

Figure 1 is an isometric view of the novel decontamination bag of the present invention;
Figure 2 is a cross-sectional view of the novel decontamination bag of the present invention as seen along the line 2-2 in Figure 1;
Figure 3 is a cross-sectional view of an alternate embodiment of the novel decontamination bag of the present invention;

Figure 4 is a cross-sectional view of another embodiment of the novel decontamination bag of the present invention; and
Figure 5 is an isometric view of an additional embodiment of the novel decontamination bag of the present invention.

## DESCRIPTION OF THE PREFERRED EMBODIMENT

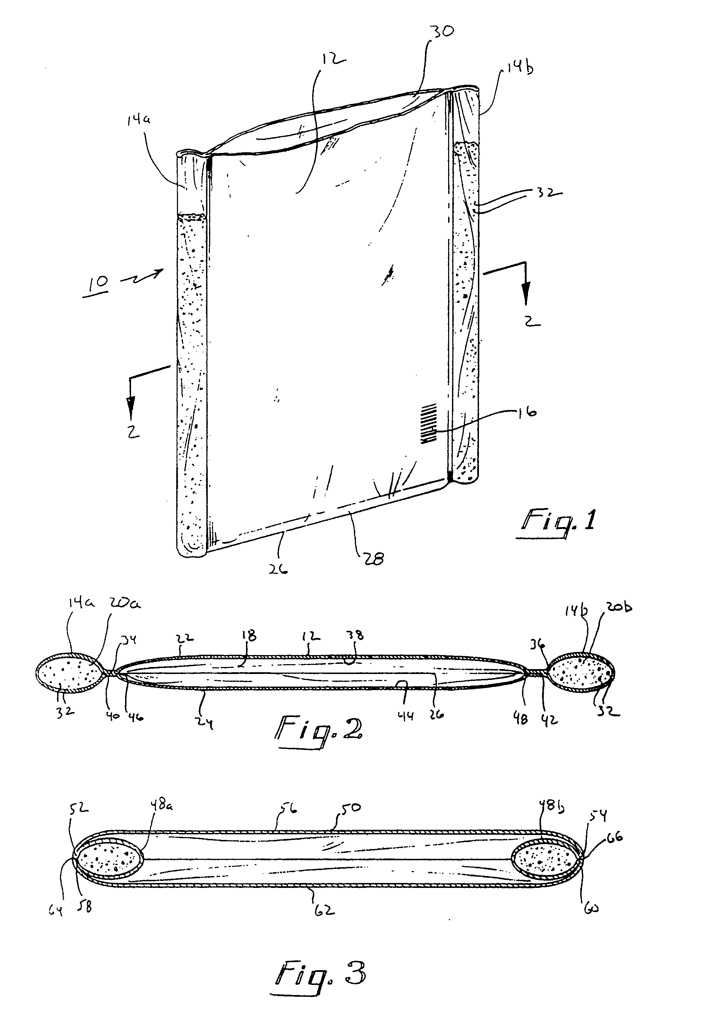

Referring initially to Figure 1, a bag, generally designated 10 for use in sterilizing infectious waste is shown to comprise a container 12 and pouches 14a and 14b. Figure 1 shows that bag 10 may be visually coded, such as by placing a bar code 16 on container 12, to identify the bag 10 as an infectious waste bag and to indicate whether bag 10 includes a dry granulated medium 32 which has disinfectant constituents. Alternatively, a phosphorescent substance (not shown) may be disposed on bag 10 in place of or in addition to bar code 16 in order to classify bag 10. Additionally, bag 10 may be color coded to properly identify and classify bag 10. For example, through bag 10 must be red or rust to signify it contains infectious waste, the shade of coloring may be such as to be properly detectable for accurate identification. Finally, bag 10 is preferably made of a lightweight, flexible, yet strong material, such as a plastic material or any of the well-known biodegradable materials which are widely used in the fabrication of bags.

The details of bag 10 are perhaps best seen in Figure 2, where it can be seen that container 12 forms a void 18, and pouches 14a and 14b form cavities 20a, 20b, respectively. More specifically, container 12 comprises a first side 22 and a second side 24. Sides 22 and 24 are joined at bottom seam 26, shown in Figure 2, to establish closed end 28 of container 12, shown in Figure 1. Sides 22 and 24 are joined at bottom seam 26 by any means, such as bonding, which establishes a fluid tight seal at bottom seam 26. Alternatively, sides 22 and 24 could be formed from a single sheet of material (not shown) in which case bottom seam 26 would be replaced by a fold in the single sheet. On the other hand, sides 22 and 24 of container 12 are not joined at open end 30 of container 12. Thus, infectious waste may be placed into void 18 of container 12 through open end 30. Importantly, open end 30 is closeable, such as by encircling open end 30 with a string or tie-wrap (not shown) and then drawing sides 22, 24 together by tightening the string or tie-wrap. Accordingly, the passage of infectious waste out of container 12 through end 30 may be prevented by appropriately closing end 30. Alternatively, after infectious waste has been deposited in container 12, sides 22, 24 may be sealed together at open end 30 to thereby close end 30.

As disclosed above, pouches 14a, 14b form enclosed cavities 20a, 20b, respectively. A medium 32 that contains a disinfectant constituent may be held in each of the cavities 20a and 20b. As will be appreciated, pouches 14a, 14b may be attached to or formed with container 12 in a variety of ways, as long as the medium 32 which is held within the cavities 20a, 20b remains separate from the infectious waste that is held within void 18 of container 12 until bag 10 is destroyed. Figures 2, 3, 4, and 5 show various alternate embodiments of bag 10, each of which embodiments uses a particular structure for establishing pouch 14. First, Figure 2 shows an embodiment of bag 10 wherein pouches 14a, 14b are integrally formed with container 12 by bonding portions 34, 36, respectively of inside wall 38 of side 22 to respective portions 40, 42 of inside wall 44 of side 24. A first fluid tight seam 46 is accordingly formed between portions 34, 40, while a second fluid tight seam 48 is formed between portions 36, 42. As shown in Figure 2, the seams 46, 48 separate container 12 from pouches 14a, 14b, respectively.

Figure 3 also shows an embodiment of bag 10 wherein pouches 48a and 48b are formed integrally with a container 50. In contrast to the structure shown in Figure 2, however, the embodiment of bag 10 shown in Figure 3 establishes the pouches 48a, 48b by a different combination of structure. More specifically, portions 52, 54 of outside wall 56 of container 50 are respectively bonded to portions 58, 60 of outside wall 62. Accordingly, a first fluid tight seam 64 is formed between portions 52, 58, while a second fluid tight seam 66 is formed between portions 54, 60. As shown in Figure 3, the seams 64, 66 separate container 50 from pouches 48a and 48b, respectively.

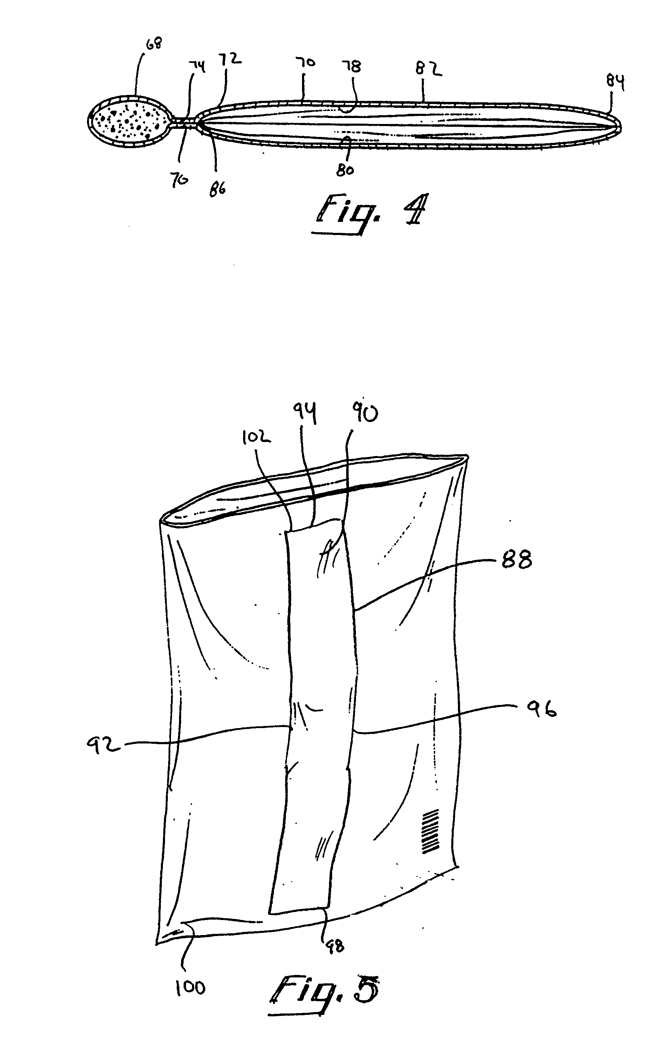

Figure 4 shows yet an additional embodiment of bag 10 wherein a pouch 68 is formed integrally with a container 70. In contrast to the embodiments of bag 10 shown in Figures 2 and 3 however, Figure 4 shows that pouch 68 may be formed along only one edge 72 of container 70 by bonding together portions 74, 76 of inside walls 78, 80, respectively, of respective sides 82, 84. Thus, a fluid tight seam 86 is formed between portions 74, 76 to separate pouch 68 from container 70.

In Figure 5, a pouch 88 is shown to comprise a separate sheet 90 which may be bonded at its edges 92, 94, 96, 98 to any convenient location of container 100. Importantly, sheet 90 is not drawn tightly across container 100, but is relatively flaccid. Accordingly, an enclosed cavity 102 is formed between the sheet 90 and the container 100 for holding a disinfectant-containing medium (not shown in Figure 5) within cavity 102 of pouch 88.

As disclosed above, the medium 32 which is contained in pouches 14a, 14b, shown in Figure 2, comprises a predetermined amount of disinfectant constituent. For example, medium 32 may contain granulated or powdered sodium hypochlorite, or another appropriate disinfectant. In addition, medium 32 may also comprise a pigment constituent which, when mixed with liquid, forms a dye for staining the waste held inside container 12. Medium 32 may also contain acidity (i.e., pH) adjusting constituents for appropriately establishing the acidity of a solution in which bag 10 may be destroyed. Additionally, medium 32 may contain a deodorant constituent, as well as surfactants. Surfactants, when mixed with water during the waste decontamination process described below, prevent the water from beading. Specifically, to ensure thorough mixing of the disinfectant solution with the waste, it is desireable that the solution does not form beads over parts of the destroyed waste while not adhering to other parts of the waste. Lastly, medium 32 may contain sodium carbonate and inert filler material.

## OPERATION

In its operation, reference is made to Figures 1 and 2. After properly identifying bag 10 as an infectious waste disposal bag having an associated disinfectant medium 30, such as, for example, by means of bar code 16, infectious waste may be placed through open end 30 into void 18 of container 12. When it is desired to dispose of bag 10, open end 30 is closed or sealed to prevent infectious waste from spilling out of bag 10. More specifically, open end 30 may be closed by sealing side 22 to side 24 at end 30. For example, side 22 could be solvent bonded to side 24 or heat sealed against side 24. Alternatively, side 22 may be held against side 24 at end 30 by, for example, encircling end 30 with a drawstring (not shown) and then drawing side 22 against side 24 by tightening the drawstring around end 30. Bag 10 is then transported to an appropriate disposal unit (not shown), in which bag 10 is mechanically destroyed, as by shredding, pulverizing, blending, or the like.

It is to be understood that as bag 10 and its contents are destroyed, the contents of bag 10 will typically be mixed together. Further, bag 10 and the contents of bag 10 may be wetted and heated during the destruction process. Thus, during the destruction process, pouches 14a, 14b are destroyed with bag 10 and the disinfectant constituents of medium 32 are thereby released from pouches 14a, 14b and onto the infectious waste contained in bag 10. Because bag 10 and its contents are wetted during the destruction process, the disinfectant constituents mix with the water to form a disinfectant solution which intimately contacts the waste and thereby sterilizes the waste. Thus, the waste, the disinfectant solution, and the remains of bag 10 are effectively thoroughly mixed in a liquid slurry as bag 10 is destroyed. Accordingly, the infectious waste which was contained in bag 10 is sterilized, or decontaminated, during the process disclosed above.

In addition to the decontamination process which occurs during the destruction of bag 10, the pigment constituents which are contained in medium 32 are also released from pouches 14a and 14b when bag 10 is destroyed. Accordingly, the pigment constituents enter the disinfectant solution and thus contact the infectious waste that is being sterilized to thereby stain the waste. After the destruction process, the pigment stain on the remnants of the formerly infectious waste indicates that the waste has undergone the sterilization process.

While the particular bag with disinfectant for sterilizing infectious waste as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

## Claims

1. A bag for use in the decontamination of infectious waste, which comprises:

    means for surroundingly holding said waste; and

    means for attaching a medium having disinfectant constituents to said holding means for release of said medium onto said waste when said bag is destroyed.

2. A bag for use in the decontamination of infectious waste according to claim 1, characterised in that said holding means is a container, said container having a closed end and a closeable open end.

3. A bag for use in the decontamination of infectious waste according to claim 1 or 3, characterised by further comprising means for sealing said open end of said container.

4. A bag for use in the decontamination of infectious waste according to any one of claims 1 to 3, characterised in that said attaching means is a pouch, said pouch forming a cavity for holding said medium.

5. A bag for use in the decontamination of infectious waste according to claim 4, characterised in that said pouch is formed integrally with said container.

6. A bag for use in the decontamination of infectious waste according to claim 5, characterised in that said container forms a void, said container comprising a first side and a second side oppositely disposed across said void, said pouch being formed by attaching a portion of said first side to a portion of said second side.

7. A bag for use in the decontamination of infectious waste according to claim 6, characterised in that said portion of said first side is bonded to said portion of said second side to create a seam separating said container from said pouch.

8. A bag for use in the decontamination of infectious waste according to any one of claims 1 to 5, characterised in that said pouch comprises a sheet of material having an edge, said edge being attached to said container to form an enclosed cavity therebetween for holding said medium.

9. a bag for use in the decontamination of infectious waste according to any one of the preceding claims, characterised in that a visual code is disposed on said container to identify said container.

10. A bag for use in the decontamination of infectious waste according to any one of the preceding claims, wherein said disinfectant comprises sodium hypochlorite.

11. A bag for use in the decontamination of infectious waste according to any of claims 1 to 8, characterised in that said disinfectant is wetted to form a solution comprising chlorine dioxide.

12. A method for manufacturing a bag for use in the decontamination of infectious waste, which comprises:

    forming a container with a closed end and a closeable open end;

    enclosing a medium having disinfectant constituents in a pouch; and

    attaching said pouch to said container for release of said medium onto said waste when said bag is destroyed.

13. A method for manufacturing a bag for use in the decontamination of infectious waste according to claim 12, characterised in that said container forms a void, said container comprising a first side and a second side oppositely disposed across said void, said pouch being formed by attaching a portion of said first side to a portion of said second side.

14. A method for manufacturing a bag for use in the decontamination of infectious waste according to claim 13, characterised in that said pouch is formed by bonding said portion of said first side to said portion of said second side to create a seam separating said container from said pouch.

*Fig.1*

*Fig.2*

*Fig. 3*

_Fig. 4_

_Fig. 5_